# EUROPEAN PATENT SPECIFICATION

(11) **EP 4 324 502 B1**
(45) Date of publication and mention of the grant of the patent: **22.04.2026**
(21) Application number: 23219602.2
(22) Date of filing: 09.03.2018
(51) Int. Cl.: A61F 9/00, A61F 9/007, A61M 5/158, A61M 5/315, A61M 5/32

(54) **INTRAOCULAR SHUNT INSERTER**
INTRAOKULARE SHUNT-EINSETZER
DISPOSITIF D'INSERTION D'UN SHUNT INTRAOCULAIRE

(43) Date of publication of application: 21.02.2024
(62) Divisional of application: 18908302.5
(73) Proprietor: Aquesys, Inc., North Chicago, IL 60064 (US)
(72) Inventor: ROMODA, Laszlo O., Irvine, 92612 (US); HORVATH, Christopher, Mission Viejo, 92691 (US)
(74) Representative: Cohausz & Florack

(56) References cited:
- US-A1- 2015 133 946

## Description

### BACKGROUND

Glaucoma is a disease of the eye that affects millions of people. Glaucoma is associated with an increase in intraocular pressure resulting either from a failure of a drainage system of an eye to adequately remove aqueous humor from an anterior chamber of the eye or overproduction of aqueous humor by a ciliary body in the eye. Build-up of aqueous humor and resulting intraocular pressure may result in irreversible damage to the optic nerve and the retina, which may lead to irreversible retinal damage and blindness.

Glaucoma may be treated in a number of different ways. One manner of treatment involves delivery of drugs such as beta-blockers or prostaglandins to the eye to either reduce production of aqueous humor or increase flow of aqueous humor from an anterior chamber of the eye. Glaucoma filtration surgery is a surgical procedure typically used to treat glaucoma. The procedure involves placing a shunt in the eye to relieve intraocular pressure by creating a pathway for draining aqueous humor from the anterior chamber of the eye. The shunt is typically positioned in the eye such that it creates a drainage pathway between the anterior chamber of the eye and a region of lower pressure. Such fluid flow pathways allow for aqueous humor to exit the anterior chamber.

US 2015/133946 A1 discloses a system for deploying an intraocular shunt according to the preamble of claim 1.

### SUMMARY

The importance of lowering intraocular pressure (IOP) in delaying glaucomatous progression is well documented. When drug therapy fails, or is not tolerated, surgical intervention is warranted. There are various surgical filtration methods for lowering intraocular pressure by creating a fluid flow-path between the anterior chamber and the subconjunctival tissue. In one particular method, an intraocular shunt is implanted with an inserter by directing a needle, which holds the shunt through the cornea, across the anterior chamber, and through the trabecular meshwork and sclera, and into the subconjunctival space. See, for example, U.S. Patent No. 6,544,249, U.S. Patent Application Publication No. 2008/0108933, and U.S. Patent No. 6,007,511, which are referred to herein.

Existing inserters may have components that move inadvertently and may not always provide desired levels of precision and feedback during a procedure. During a procedure, an operator may not be able to differentiate between the different stages of the insertion process, such as shunt insertion and needle retraction. This may require the operator to manually and/or visually review steps of the procedure, which increases the time that a careful and attentive operator must devote to each step of the procedure. As such, this can increase surgery time, potentially cause greater trauma to the patient, and nevertheless be reliant on tactile or visual perception of components without certainty that certain milestones or positions have been achieved.

Accordingly, the present disclosure contemplates these problems, provides solutions to these problems, and relates to the realization that precision can be increased while reducing operator effort and surgery time, in some embodiments, implementing certain advantageous features in a shunt inserter.

The present invention is defined by appended claim 1. Preferred embodiments of the invention are defined by the dependent claims.

Some embodiments disclosed herein provide an intraocular shunt inserter having an actuator that that permits the operator to deliver and/or release an intraocular shunt. The inserter can be configured to provide a frictional track or resistance against which an operator can move the actuator, whether sliding or rotating. This resistance to movement can ensure that the inserter exposes or releases the shunt only when intended by the operator. Further, the resistance can tend to cause the operator to operate the inserter using a greater degree of precision and control.

Optionally, some embodiments can comprise one or more feedback components that can serve as indicators of motion or completion of steps in the procedure. For example, the inserter can comprise an actuator, whether sliding or rotating, that can provide one or more audible clicks and/or barriers of increased resistance that can serve as signals to the operator that a certain position or step of the procedure has been completed. In some embodiments, a slider component can contact against a first engagement structure or indicator on the inserter to create an audible click or barrier of increased resistance. Continued movement beyond the click or barrier of increased resistance can allow the operator to move the slider component towards a second, third, fourth, or other engagement structure or indicator that can create an audible click or barrier of increased resistance to signal to the operator that the slider component has been advanced to a predetermined location and/or that additional positions or steps of the procedure have been completed. Accordingly, the inserter can advantageously provide improved precision and feedback to an operator.

Further, some embodiments disclosed herein can optionally provide an inserter with a bended shaft or needle that can provide greater tactile control of the inserter and improved clearance during a procedure. The needle can extend from a distal end portion of the inserter and comprise a bend at which a longitudinal axis of the needle is redirected along a different axis. The bend can enable an operator to more easily manipulate and/or perceive the position of a bevel of the needle during the procedure. Thus, some embodiments can advantageously permit an operator to more easily visually verify that a certain result has been achieved. For example, by rotating the bevel of the needle, an operator can "tent" the conjunctiva of the eye, thereby facilitating placement and delivery of the intraocular shunt into a subconjunctival target location. Further, the bend can cause a longitudinal axis of a housing of the inserter to be spaced at a greater distance apart from a patient's face during the procedure than compared to straight-needle-type inserters.

For example, an inserter can include a housing and a slider component. The housing include a distal portion, a proximal portion, a longitudinal axis extending between the distal and proximal portions, an interior cavity, and an elongate slot extending along an outer surface of the housing into the cavity. The slider component can be coupled to the housing and positioned along the outer surface of the housing. The slider component can be slidable along the elongate slot to operate the inserter. The slider component can include a guide tab disposed within the guide channel of the housing body. The slider component can also include a friction tab with a biasing member configured to urge against the housing body to urge the guide tab against the channel wall of the guide channel.

An operator can operate the inserter by urging the slider component along an axis of the inserter. The slider component can actuate a deployment mechanism of the inserter to deliver and release an intraocular shunt. In order to do so, the operator must overcome an initial friction force provided by a friction tab of the slider component against the housing. The operator can use the slider component to advance a plunger of the inserter to urge the shunt within a lumen of the needle.

During operation of the inserter, an operator can receive tactile or horrible feedback from engagement structures of the housing, for example, as the friction tab moves across an engagement structure. The feedback can correspond to the position of the shunt relative to the needle within the inserter. The feedback can be provided by a discontinuity on the housing.

For example, in some embodiments, the inserter can generate an audible signal using a biasing member configured to engage a discontinuity of the housing body. The audible or tactile signal can indicate a position of the slider component relative to the inserter and/or indicate a position of the shunt or stage of shunt delivery.

### BRIEF DESCRIPTION OF THE DRAWINGS

The accompanying drawings, which are included to provide further understanding of the subject technology and are incorporated in and constitute a part of this specification, illustrate aspects of the disclosure and together with the description serve to explain the principles of the subject technology.
Figure 1A is a schematic view of a procedure for implanting an intraocular shunt into an eye using an inserter, according to some embodiments.
Figure 1B is a perspective view of an inserter for implanting an intraocular shunt into an eye, according to some embodiments.
Figure 2 is a perspective, exploded view of the inserter shown in Figure 1B, according to some embodiments.
Figure 3 is a perspective, exploded view of a drive assembly of the inserter shown in Figure 1B, according to some embodiments.
Figures 4A-4C illustrate a slider component of the inserter shown in Figure 1B, according to some embodiments.
Figure 5 is a cross-sectional view of an inserter for implanting an intraocular shunt into an eye, according to some embodiments.
Figures 6A-6C are cross-sectional views of engagement structures of the inserter, according to some embodiments.
Figure 7A is a perspective view of a sleeve mount of the drive assembly shown in Figure 3, having a straight shaft, according to some embodiments.
Figure 7B is a perspective view of a sleeve mount of the drive assembly shown in Figure 3, having a bended shaft, according to some embodiments.
Figure 8A is a perspective view of an inserter having an alignment guide for providing a bend in the shaft of the inserter, according to some embodiments.
Figure 8B is a perspective view of an alignment guide coupled to a sleeve, according to some embodiments.
Figure 8C is a side view of an alignment guide coupled to a sleeve, according to some embodiments.
Figure 9A is a front perspective view of another alignment guide, according to some embodiments.
Figure 9B is a rear perspective view of the alignment guide of Figure 9A, according to some embodiments.
Figure 10A is a perspective view of an alignment guide coupled to a sleeve with a protective cap, according to some embodiments.
Figure 10B is a side, cross-sectional view of a bevel protection device received within a needle lumen of an inserter, according to some embodiments.

### DETAILED DESCRIPTION

In the following detailed description, numerous specific details are set forth to provide a full understanding of the subject technology. It should be understood that the subject technology may be practiced without some of these specific details. In other instances, well-known structures and techniques have not been shown in detail so as not to obscure the subject technology.

Glaucoma is a disease in which the optic nerve is damaged, leading to progressive, irreversible loss of vision. It is typically associated with increased pressure of the fluid (i.e., aqueous humor) in the eye. Untreated glaucoma leads to permanent damage of the optic nerve and resultant visual field loss, which can progress to blindness. Once lost, this damaged visual field cannot be recovered.

In conditions of glaucoma, the pressure of the aqueous humor in the eye (anterior chamber) increases and this resultant increase of pressure can cause damage to the vascular system at the back of the eye and especially to the optic nerve. The treatment of glaucoma and other diseases that lead to elevated pressure in the anterior chamber involves relieving pressure within the anterior chamber to a normal level.9

Glaucoma filtration surgery is a surgical procedure typically used to treat glaucoma. The procedure involves placing a shunt in the eye to relieve intraocular pressure by creating a pathway for draining aqueous humor from the anterior chamber of the eye. The shunt is typically positioned in the eye such that it creates a drainage pathway between the anterior chamber of the eye and a region of lower pressure. Various structures and/or regions of the eye having lower pressure that have been targeted for aqueous humor drainage include Schlemm's canal, the subconjunctival space, the episcleral vein, the suprachoroidal space, the intra-Tenon's adhesion space, and the subarachnoid space. Shunts may be implanted using an ab externo approach (e.g., entering through the conjunctiva and inwards through the sclera) or an ab interno approach (e.g., entering through the cornea, across the anterior chamber, through the trabecular meshwork and sclera). For example, ab interno approaches for implanting an intraocular shunt in the subconjunctival space are shown for example in Yu et al. (U.S. Patent No. 6,544,249 and U.S. Patent Application Publication No. 2008/0108933) and Prywes (U.S. Patent No. 6,007,511), which are referred to herein.

Some methods can involve inserting into the eye a hollow shaft configured to hold an intraocular shunt. In some embodiments, the hollow shaft can be a component of a deployment device that may deploy the intraocular shunt. The hollow shaft can be coupled to a deployment device or be part of the deployment device itself. The deployment devices can include devices such as those as described in co-owned U.S. Patent No. 9,585,790, U.S. Patent No. 8,721,792, U.S. Patent No. 8,852,136, and U.S. Patent Application Publication No. 2012/0123434, filed on November 15, 2010, which are referred to herein.

As noted above, conventional deployment devices or inserters may not provide desired levels of precision and feedback, requiring additional operator effort and surgical time. The present disclosure provides various embodiments of methods and devices that can enable an operator to implant a shunt using an inserter with improved comfort, feedback and precision while reducing surgical time. As used herein, the term "shunt" includes hollow microfistula tubes similar to the type generally described in U.S. Pat. No. 6,544,249 as well as other structures that include one or more lumens or other flow paths therethrough.

In accordance with some embodiments, the inserter can be advanced into the eye via an ab-interno or an ab-externo approach. Thereafter, the shunt can be deployed from the shaft into the eye such that the shunt forms a passage from the anterior chamber into an area of lower pressure, such as Schlemm's canal, the subconjunctival space, the episcleral vein, the suprachoroidal space, the intra-Tenon's adhesion space, the subarachnoid space, or other areas of the eye. The hollow shaft is then withdrawn from the eye. Methods for delivering and implanting bioabsorbable or permanent tubes or shunts, as well as implantation devices for performing such methods, are generally disclosed in applicant's applications, including U.S. Patent Application Publication Nos. 2012/0197175, 2015/0011926, and 2016/0354244, U.S. Patent Application No. 15/613,018, as well as in U.S. Pat. Nos. 6,007,511, 6,544,249, 8,852,136, and 9,585,790.

Some methods can be conducted by making an incision in the eye prior to insertion of the deployment device. However, in some instances, the method may be conducted without making an incision in the eye prior to insertion of the deployment device. In some embodiments, the shaft that is connected to the deployment device has a sharpened point or tip. In some embodiments, the hollow shaft is a needle. Exemplary needles that may be used are commercially available from Terumo Medical Corp. (Elkington, Md). In some embodiments, the needle can have a hollow interior and a beveled tip, and the intraocular shunt can be held within the hollow interior of the needle. In some embodiments, the needle can have a hollow interior and a triple ground point or tip.

Some methods can be conducted without needing to remove an anatomical portion or feature of the eye, including but not limited to the trabecular meshwork, the iris, the cornea, or aqueous humor. Some methods can be conducted without inducing substantial ocular inflammation, such as subconjunctival blebbing or endophthalmitis. Some methods can be achieved using an ab interno approach by inserting the hollow shaft configured to hold the intraocular shunt through the cornea, across the anterior chamber, through the trabecular meshwork, and into the intra-scleral or intra-Tenon's adhesion space. However, some methods may be conducted using an ab externo approach.

In some methods conducted using an ab interno approach, the angle of entry through the cornea can be altered to affect optimal placement of the shunt in the intra-Tenon's adhesion space. The hollow shaft can be inserted into the eye at an angle above or below the corneal limbus, in contrast with entering through the corneal limbus. For example, the hollow shaft can be inserted from about 0.25 mm to about 3.0 mm above the corneal limbus. The shaft can be inserted from about 0.5 mm to about 2.5 mm above the corneal limbus. The shaft can also be inserted from about 1.0 mm to about 2.0 mm above the corneal limbus, or any specific value within any of these ranges. For example, the hollow shaft can be inserted above the corneal limbus at distances of about: 1.0 mm, 1.1 mm, 1.2 mm, 1.3 mm, 1.4 mm, 1.5 mm, 1.6 mm, 1.7 mm, 1.8 mm, 1.9 mm, or 2.0 mm.

Further, in some embodiments, placement of the shunt farther from the limbus at the exit site, as provided by an angle of entry above the limbus, can provide access to more lymphatic channels for drainage of aqueous humor, such as the episcleral lymphatic network, in addition to the conjunctival lymphatic system. A higher angle of entry also results in flatter placement in the intra-Tenon's adhesion space so that there is less bending of the shunt.

As discussed in U.S. Patent No. 8,852,136, which is referred to herein, in some embodiments, to ensure proper positioning and functioning of the intraocular shunt, the depth of penetration into the intra-Tenon's adhesion space may be important when performing some methods.

In some methods, the distal tip of the hollow shaft can pierce the sclera and intra-Tenon's adhesion space without coring, removing or causing major tissue distortion of the surrounding eye tissue. The shunt is then deployed from the shaft. Preferably, a distal portion of the hollow shaft (as opposed to the distal tip) completely enters the intra-Tenon's adhesion space before the shunt is deployed from the hollow shaft.

In accordance with some embodiments, the hollow shaft can comprise a flat bevel needle, such as a needle having a triple-ground point. The tip bevel can first pierce through the sclera and into the intra-Tenon's adhesion space by making a horizontal slit. In some methods, the needle can be advanced even further such that the entire flat bevel penetrates into the intra-Tenon's adhesion space, to spread and open the tissue to a full circular diameter.

Further, in accordance with an aspect of some methods, the intra-Tenon's channel can be urged open by the flat bevel portion of the needle so that the material around the opening is sufficiently stretched and a pinching of the shunt in that zone is avoided, thus preventing the shunt from failing due to the pinching or constriction. Full entry of the flat bevel into the intra-Tenon's adhesion space causes minor distortion and trauma to the local area. However, this area ultimately surrounds and conforms to the shunt once the shunt is deployed in the eye.

In some embodiments, the inserter can function as a one-handed device in order to allow an operator to keep their other hand on a fixation device that holds the eye, such as a hook. This can improve surgical control and placement accuracy and makes the surgery easier as well.

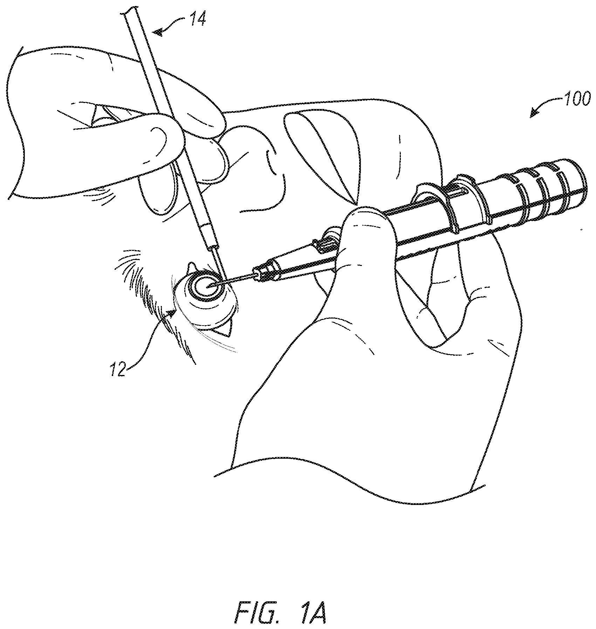

An illustration of a procedure for treating an eye 12 is shown in Figure 1A. Figure 1A illustrates the use of a hook 14 for holding the eye 12 and an inserter 100 for introducing an intraocular shunt into the eye.

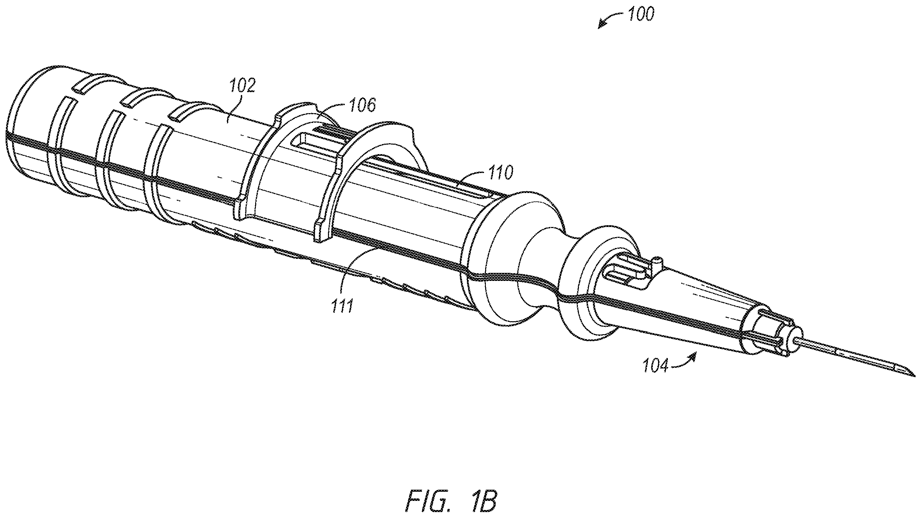

Figures 1B-9 illustrate further details of the inserter 100 shown in Figure 1A. The inserter 100 can be actuated using a single hand, thus facilitating use of the inserter by an operator. The inserter 100 can comprise a housing 102, a needle assembly 104, and a slider component 106. As shown in Figure 1B, the inserter 100 can be configured such that the slider component 106 is coupled to the housing 102 via guide channels 111 and slidable along an elongate slot 110 of the housing 102. The slider component 106 can be selectively movable by an operator in order to actuate movement of components of the needle assembly 104.

For example, when the slider component 106 moves distally along the slot 110 (i.e., in a direction toward the needle assembly 104), the slider component 106 can result in or cause a shunt (not shown) to be advanced within the needle assembly 104, and in some embodiments, released from the needle assembly 104. In accordance with some embodiments discussed further herein, movement of the slider component 106 can result in translational and/or rotational movement of components of the needle assembly 104. The sliding movement of the slider component 106 can be converted into rotational movement, which can thereafter be converted to movement along a longitudinal axis of the inserter 100. One of the benefits of this innovative and complex movement-conversion mechanism is that it enables embodiments of the inserter to provide precise, measured movements of its components within a compact assembly.

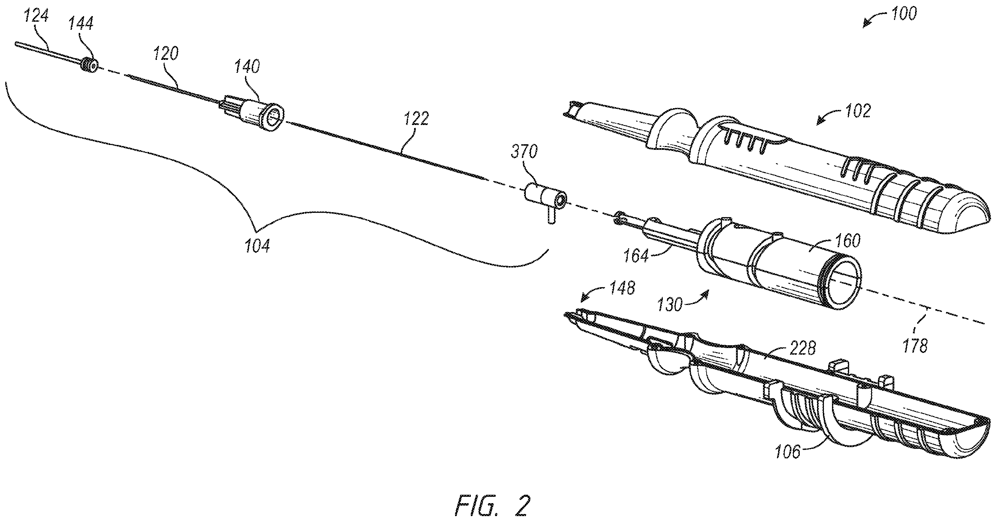

As illustrated in Figure 2, the needle assembly 104 can comprise a needle component 120, a plunger 122, and a sleeve component 124. The needle component 120 can comprise a 25 GA or 27 GA needle. The plunger 122 can be slidably movable within a lumen of the needle component 120 along a longitudinal axis 178 of the inserter 100. Further, the needle component 120 can be slidably movable within a lumen of the sleeve component 124 along the longitudinal axis 178. Each of the needle component 120 and the plunger 122 can be coupled to respective drive components of a drive assembly 130 disposed within the housing 102. When in the assembled state, the inserter 100 can be configured such that the needle component 120, the plunger 122, and the sleeve component 124 are aligned along or coaxial with the longitudinal axis 178. Some drive assemblies for actuating a plunger and for withdrawing a needle of an inserter are disclosed in U.S. Patent Application Nos. 13/336,803, 12/946,645, 12/620,564, 12/946,653, 12/946,565, and 11/771,805 and U.S. Patent No. 9,585,790, which are referred to herein.

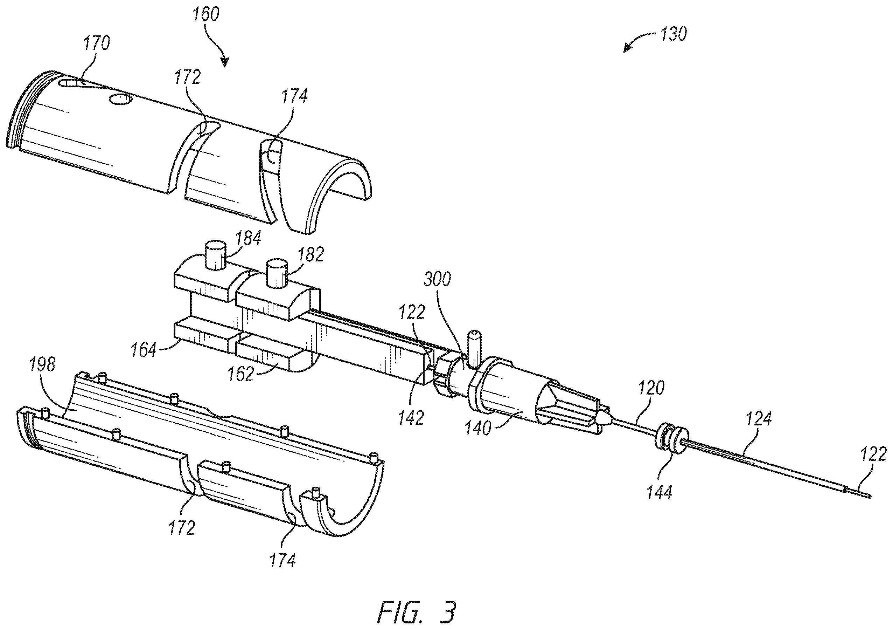

Referring to Figures 2 and 3, the needle component 120, the plunger 122, and the sleeve component 124 can be operably coupled to the drive assembly 130 and/or the housing 102. For example, the needle component 120 can be coupled to a needle mount 140. The needle mount 140 can be fixedly coupled to a proximal end portion of the needle component 120 such that rotational and longitudinal movement between the needle component 120 and the needle mount 140 is restricted or prevented. The needle mount 140 can be enclosed within a distal end portion of the housing 102 when the inserter 100 is assembled. Further, as illustrated in Figure 3 and discussed further below, the needle mount 140 can be coupled to a needle driver 164 (and in the illustrated embodiment, via a rotational adjustment component 300) of the drive assembly 130.

Further, as shown in Figure 3, the plunger 122 can be coupled to a plunger mount 142. The plunger mount 142, can be fixedly coupled to a proximal end portion or midsection of the plunger 122 to restrict or prevent rotational and longitudinal movement of the plunger 122 relative to the plunger mount 142. Further, as illustrated in Figure 3 and discussed further below, the plunger mount 142 can be coupled to a plunger driver 162 of the drive assembly 130.

Furthermore, the sleeve component 124 can be coupled to a sleeve mount 144. The sleeve mount 144 can be coupled to a proximal end portion of the sleeve component 124 so as to prevent rotational and longitudinal movement between the sleeve component 124 and the sleeve mount 144. The sleeve mount 144 can be coupled to a portion 148 of the housing 102, as discussed below.

As noted above, the needle component 120, the plunger 122, and the sleeve component 124 can be operably coupled to the drive assembly 130 and/or the housing 102. Such coupling can occur via the needle mount 140, the plunger mount 142, and the sleeve mount 144. In turn, the needle mount 140, the plunger mount 142, and the sleeve mount 144 can be coupled to one or more drive components that engage with the drive assembly 134 to the housing 102.

In accordance with some embodiments, the drive assembly 130 can be coupled to the needle component 120 and the plunger 122 to actuate movement along the longitudinal axis 178 of the needle component 120 and the plunger 122 relative to the housing 102. For example, the drive assembly 130 can be configured to rotate or slide within the housing 102. The drive assembly 130 can transfer a longitudinal or axial force along the longitudinal axis 178 to the needle component 120 and/or the plunger 122, independently or at the same time, to result in movement of the needle component 120 and the plunger 122 relative to the housing 102 along the longitudinal axis 178.

As discussed herein, motion of the slider component 106 can result in motion of the drive assembly 130 and thereby result in motion of components of the drive assembly 130 relative to the housing 102. Some embodiments can be configured such that the slider component 106 can be longitudinally movable or slidable along the longitudinal axis 178 relative to the housing 102 in order to drive or result in linear motion of the needle component 120 and the plunger 122 and consequently a shunt.

As shown in Figure 3, the drive assembly 130 can comprise a drive component 160, a plunger driver 162, and a needle driver 164. In some embodiments, longitudinal or linear motion of the slider component 106 along the longitudinal axis 178 can be converted to result in rotation of the drive component 160 of the drive assembly 130, which can then be converted to result in longitudinal or linear motion of the needle component 120 and the plunger 122 along the longitudinal axis 178 relative to the housing 102. In accordance with some embodiments, motion of the components along the longitudinal axis 178 can be parallel relative to the longitudinal axis 178.

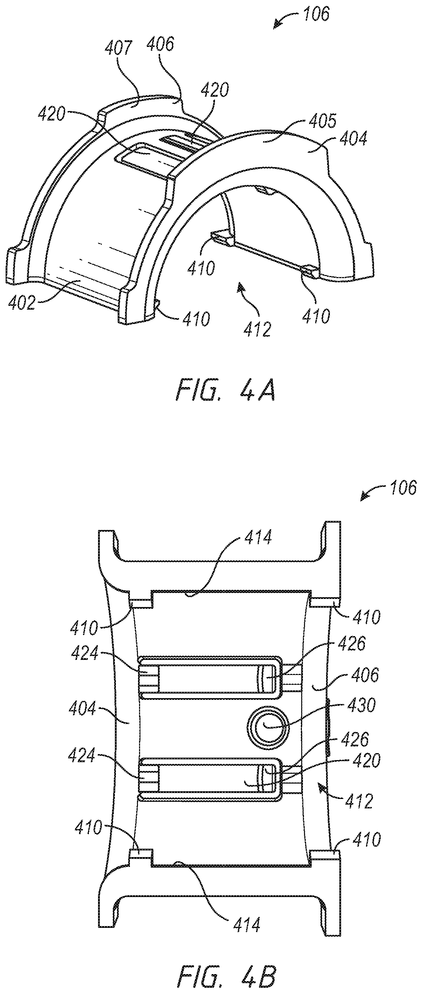

Figure 3 also illustrates an embodiment of the drive component 160. The drive component 160 can comprise a groove 170 that can be configured to engage with a corresponding protrusion (not shown) of the slider component 106. Further, the drive component 160 can also comprise first and second driving grooves 172, 174 that can be configured to slidingly engage corresponding protrusions of the plunger driver 162 and the needle driver 164. Thus, the slider component 106 can comprise a protrusion 430 (shown in Figure 4B), the plunger driver 162 can comprise a protrusion 182, and the needle driver 164 can comprise a protrusion 184. This arrangement of slots and protrusions can facilitate the transfer of motion from the slider component 106 to the respective ones of the needle component 120 and the plunger 122. Further, the plunger driver 162 and the needle driver 164 can comprise rounded bodies that contact and slide against an inner guide surface 198 of the drive component 160 when seated within the drive component 160.

Figures 4A-4C illustrate the slider component 106 of the inserter 100 shown in Figure 1B, according to some embodiments. Figure 4A illustrates a perspective view of the slider component 106. The slider component 106 can comprise a slider body 402 with a proximal end portion 406 and a distal end portion 404. The slider body 402 can have a generally semi-cylindrical shape. The proximal end portion 406 and the distal end portion 404 can comprise a raised distal boundary or edge 405 and a raised proximal boundary or edge 407 that protrude radially from the slider component 106 in order to provide a secure, ergonomic grip with a thumb or finger of the operator during use.

The slider component 106 can comprise one or more guide tabs 410. The guide tabs 410 can be disposed at the distal end portion 404 and the proximal end portion 406. For example, the guide tabs 410 can extend inwardly toward an interior region 412 of the slider component 106. The interior region 412 of the slider component 106 can comprise a generally semi-cylindrical shape or cavity that can be configured to be coupled to the inserter 100, such as by receiving a portion of the inserter 100 therein. When coupled to the inserter 100, the guide tabs 410 can be disposed within the guide channel 111 of the housing 102 to couple the slider component 106 to the housing 102. Thus, the guide tabs 410 of the slider component 106 can be retained within the guide channel 111, thereby restraining radial movement of the slider component 106 relative to the housing 102 while allowing for axial or longitudinal movement of the slider component 106 along the housing 102, as described herein.

Optionally, the slider component 106 can be configured to include a plurality of guide tabs 410 extending radially inwardly into the interior region 412 from opposing faces or edges of the slider component 106. For example, as illustrated in Figure 4B, the slider component 106 can comprise a pair of guide tabs 410 extending radially inwardly from interior side edges 414 of the slider component 106. The guide tabs 410 can be spaced between about 90 degrees to about 180 degrees apart from each other along the interior side edges 414 or an inner surface of the interior region 412.

Further, in some embodiments, the guide tabs 410 can be beveled to allow the slider component 106 to be pressed or snapped onto the housing 102 and into the guide channel 111. For example, one or more of the guide tabs 410 can comprise a beveled portion facing away from the interior region 412. Thus, when the slider component 106 is pressed onto the housing 102, the slider component 106 can deflect slightly to open the interior region 412 until the guide tabs 410 snap into place in the guide channels 111.

Figure 4B illustrates a bottom view of the slider component 106. Referring to Figure 4B, the protrusion 430 can be formed integrally with the body 402 of the slider component 106. However, in accordance with some embodiments, the protrusion 430 can also be formed as a separate component that is later attached to the body 402 of the slider component 106. As described herein, the motion of the slider component 106 can be transmitted to the drive assembly 130 via the protrusion 430 and thereby result in motion of the components of the drive assembly 130 relative to the housing 102. In some embodiments, the protrusion 430 can be disposed at the proximal end portion 406 of the slider component 106. In some embodiments, the protrusion 430 can be disposed at the distal end portion 404 of the slider component. In some embodiments, the protrusion 430 can be disposed in between the proximal end portion 406 and the distal end portion 404.

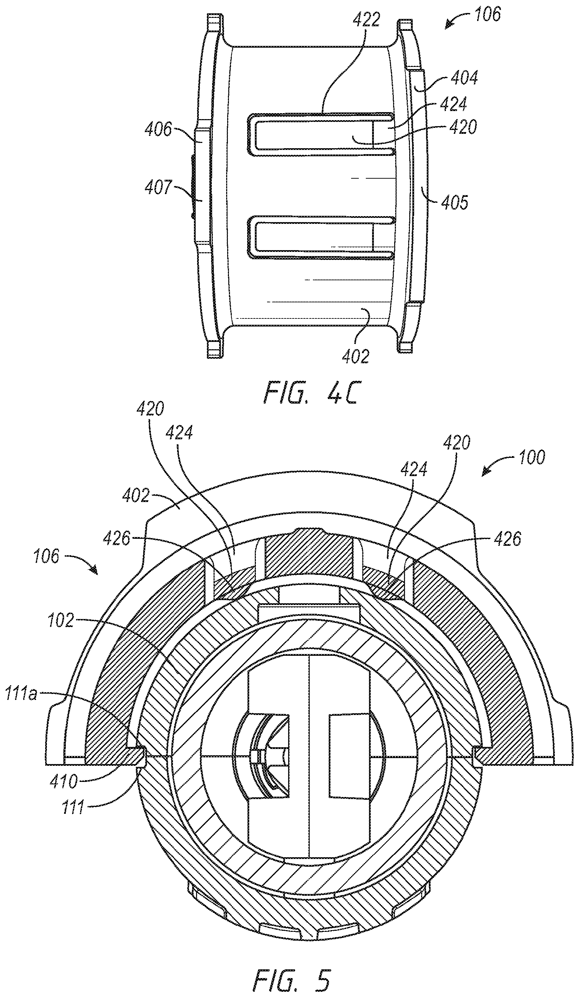

Figure 4C illustrates a top view of the slider component 106. With reference to Figures 4B and 4C, friction tabs 420 can be integrally formed with the body 402 of the slider component 106. As used herein, "integrally formed" can be defined as being formed as a single, continuous component or piece. Such components can be injection molded as a single, continuous component or begin as a single part that is later machined or otherwise processed to create various features that are coupled together from a single, continuous material. For example, through a process such as injection molding or laser beam machining, the friction tabs 420 can be formed by creating slots 422 that thereby define shape of the friction tab 420 and allow the friction tab 420 to move relative to the body 402. The friction tab 420 can be attached to the body 402, such as by a cantilevered connection or via pivots or attachment points 424. The attachment points 424 can be reinforced or include additional body material to improve cycle fatigue strength. In some embodiments, the friction tabs 420 can be formed as a separate component that are later attached to the body 402 of the slider component 106.

As shown in Figure 4B, the friction tab 420 can include a biasing member or friction protrusion 426 which extends radially beyond adjacent portions of the body 402. The protrusion 426 can extend radially inwardly toward or into the interior region 412. The protrusion 426 can be tapered or beveled in shape to allow the slider component 106 to travel over one or more engagement structures, such as the notches, serrations, slots, protrusions, or bumps, of the housing 102 in one direction and resist direction in an opposite direction.

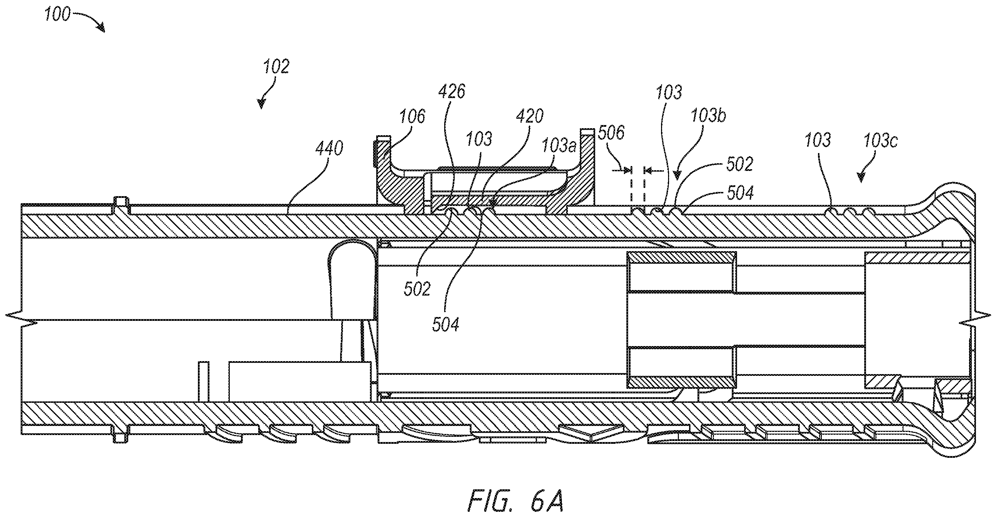

For example, the protrusion 426 can comprise a deflection-facilitating distal surface that extends at an obtuse angle with respect to an inner surface of the slider component 106 and faces the distal boundary or edge 405. As such, in some embodiments, the friction tab 420 can be moveable or deflectable relative to the body of the slider component 106, and the distal surface of the protrusion 426 can permit the protrusion 426 to begin radial deflection as it slides axially over an engagement structure formed on the housing 102. Such a configuration is illustrated in the side view of Figure 6A. The distal surface of the protrusion 426 can therefore be configured to permit or facilitate distal motion of the slider component 106 along the housing 102.

Further, the protrusion 426 can comprise an anti-reversing proximal surface that extends perpendicularly from or at an angle (e.g., if a protrusion, at an acute angle, or if a notch, at an obtuse angle) with respect to the inner surface of the slider component 106 and faces the proximal boundary or edge 407. The proximal surface of the protrusion 426 can therefore be configured to catch or restrict proximal motion of the slider component 106 along the housing 102.

In some embodiments, the engagement structure of the housing 102 can comprise a deflection-facilitating cross-sectional profile, such as a rounded shape or angled shape (e.g., extending an obtuse angle from an outer surface 440 of the housing 102) along a proximal-facing portion of the engagement structure that initially contacts the protrusion 426 as the slider component 106 is advanced distally along the housing 102. Further, in some embodiments, both the proximal-facing portion and a distal-facing portion of the engagement structure can comprise a deflection-facilitating cross-sectional profile.

Optionally, the engagement structure of the housing can comprise an anti-reversing cross-sectional profile. For example, the engagement structure can comprise an edge that extends perpendicularly or at an angle (e.g., if a protrusion, at an obtuse angle, or if a notch, at an acute angle) from the outer surface 440 of the housing 102. In some embodiments, the distal-facing portion of the engagement structure can comprise the anti-reversing cross-sectional profile. Thus, the distal-facing portion of the engagement structure can catch with or engage the proximal surface of the protrusion 426 to restrict proximal motion of the slider component 106 along the housing 102. Additionally, in some embodiments, the proximal-facing portion of the engagement structure can comprise a deflection-facilitating cross-sectional profile and the distal-facing portion of the engagement structure can comprise an anti-reversing cross-sectional profile.

Optionally, as described further herein, the protrusion 426 and/or the engagement structure can be shaped to provide audible and/or tactile feedback to the operator. As will be appreciated by personal skill in the art, a snap or click can be created by deflecting the friction tab 420 and quickly permitting release of the friction tab 420 into contact with the outer surface 440 of the housing 102. This can be accomplished in a variety of ways, including when the engagement structure includes a perpendicular portion that would permit the protrusion 426 of the friction tab 420 to rapidly move radially into contact with the outer surface 440 of the housing 102. For example, the distal-facing portion of the engagement structure can extend perpendicularly relative to the outer surface 440 of the housing 102 such that distal advancement of the slider component 106 over the engagement structure permits the protrusion 426 to snap radially inwardly against the outer surface 440 of the housing 102, thereby providing audible and/or tactile feedback to the operator.

Referring to Figure 5, an embodiment of an inserter 100 is shown with the slider component 106 attached to the housing 102 by engaging the guide tabs 410 within the guide channels 111. In some embodiments, the guide channels 111 are disposed on opposite sides of the housing 102. For example, the guide tabs 410 and/or the guide channels 111 can be oriented at different angular locations along the slider component 106 and/or the housing 102, such at about 180 degrees away from each other, less than 180 degrees away from each other, less than 170 degrees away from each other, less than 160 degrees away from each other, or less than 150 degrees away from each other.

When the slider component 106 is engaged in the guide channels 111, the protrusions 426 can contact portions of the housing 102. For example, the protrusions 426 can contact the housing 102 adjacent to the slot 110. In some embodiments, the protrusions 426 can be positioned to contact the housing 102 on opposing sides of the slot 110.

The protrusions 426 may be biased into contact with the housing 102. In some embodiments, the protrusions 426 can contact the housing 102 and cause the friction tabs 420 to be urged or deflected radially away from the housing 102, e.g., by deforming along the length of the friction tabs 420 or at the attachment points 424. In some embodiments, body of the friction tabs 420 and/or the attachment points 424 can resist this deflection or deformation, providing a reaction force via the friction tabs 420 and the protrusions 426 against the housing 102. In some embodiments, the attachment points 424 and the friction tabs 420 can be biased to provide a biasing force. This biasing force can urge the slider component 106 radially away from the housing 102, thereby causing the guide tabs 410 of the slider component 106 to be pressed against the inside of the guide channel 111. Thus, although the guide tabs 410 limit the radial outward motion of the slider component 106 relative to the housing 102, the biasing force exerted via the friction tabs 420 can increase the friction between the slider component 106 and the housing 102. Therefore, in some embodiments, the slider component 106 can tend to remain stationary along the housing 102 unless a sufficient axial force is exerted against the slider component 106 to overcome the friction between the slider component 106 on the housing 102.

For example, as shown in Figure 5, as the slider component 106 is forced radially away from the housing 102, the guide tabs 410 move toward a channel wall 111a of the guide channel 111. Therefore, in a resting state the slider component 106 is frictionally retained between the guide tabs 410 and the channel wall 111a and the protrusions 426 of the friction tabs 420 against the outer surface of the housing 102. Advantageously, this arrangement also minimizes radial play within the slider component 106 relative to the housing 102.

Additionally, by engaging the friction tabs 420 and the guide tabs 410 against the housing 102, the friction force between the slider component 106 and the housing 102 is enhanced. This can allow for the slider component 106 to be retained in a desired or initial position, and can prevent inadvertent movement of the slider component 106 during shipping and handling of the inserter 100. Therefore, to move the slider component 106 and thereby operate the inserter, the frictional force of the slider component 106 relative to the housing 102 must be overcome by a deliberate, intentional axial force exerted by the operator.

Referring to Figure 6A, the friction tabs 420 can further provide tactile and audible feedback to the operator during operation of the inserter 100. During operation, as the slider component 106 is advanced relative to the housing 102, the friction tabs 420, and more particularly, the friction protrusions 426 can pass over engagement structures 103 formed on the housing 102. Each engagement structure 103 can comprise a discontinuity in an outer surface 440 of the housing 102, such as such as a notch, serration, slot, protrusion, or bump. The engagement structures 103 can be indexed to reflect different stages of operation of the inserter 100 or positions of the slider component 106 along the housing 102 or slot 110. The housing 102 can be configured to include one or many engagement structures 103. Further, the engagement structures can be grouped together (as a single group or multiple groups) or spaced apart along the housing 102.

For example, as shown in Figures 6A-6C, the engagement structures 103 can be configured such that in order for the slider component 106 to move away from its initial position, the friction tabs 420 contact against a first engagement structure 103a (shown as a group of three engagement structures 103, although the first engagement structure 103a can also comprise just a single engagement structure 103 or two engagement structures 103) in the housing 102. Similarly, just before the slider component 106 reaches a certain location long its full travel path (e.g., halfway along the travel path or after the shunt inserter has exposed the shunt within the eye and just before continued advancement of the slider begins to retract the needle of the inserter back into the housing), the friction tabs 420 can contact against a second engagement structure 103b (shown as a group of three engagement structures 103, although the second engagement structure 103b can also comprise just a single engagement structure 103 or two engagement structures 103). Finally, the friction tabs 420 can click against a third engagement structure 103c (shown as a group of three engagement structures 103, although the third engagement structure 103c can also comprise just a single engagement structure 103 or two engagement structures 103) when the slider component 106 has advanced sufficiently to release the shunt. The feedback can be used to signal that the inserter 100 is performing a different operation, that the shunt or a portion of the inserter 100 has reached a certain position, and/or that the different operation can require a different actuation force.

Thus, the slider component 106 can move along the housing 102 and provide tactile and/or audible feedback to the operator regarding a position of the slider component 106 relative to the housing 102 and/or a position of the shunt or a stage of shunt delivery. In some embodiments, it may be advantageous to provide feedback to the operator when the shunt is initially exposed from the needle of the inserter. Further, it may also be advantageous to provide feedback to the operator when the inserter has released the shunt (which may not yet be fully exposed outside of the needle).

The type, frequency, and/or strength of tactile and/or audible signals can vary depending on the position of the slider component 106, the shunt, and/or the state of shunt delivery.

A tactile or audible signal may be provided only when certain milestones were achieved, such as initial movement of the slider component, initial shunt exposure of the shunt, a position prior to full release of the shunt (such as when the sleeve has been retracted halfway from its fully extended position), and/or reaching a final position of the slider component when the shunt is fully released and the needle is fully retracted (or other such positions, as discussed in U.S. Patent No. 9,585,790, the entirety of which is incorporated herein by reference). Further, some embodiments can be provided in which tactile feedback is provided only at certain milestones while audible feedback is provided at other milestones. For example, either one of tactile or audible feedback can be provided at the beginning stages while the other one of tactile or audible feedback is provided at the latter stages of the procedure. Further, either one of tactile or audible feedback can be provided at the beginning and at the end to mark initial and final slider component movement, while the other one of tactile or audible feedback is provided when the shunt is initially exposed and just prior to full release of the shunt. Various options and permutations of the above can be provided.

Optionally, the housing 102 can comprise a plurality of engagement structures 103 that provide a continuous, modest tactile or audible feedback to the operator to indicate that the slider component 106 is being advanced.

Therefore, in accordance with some embodiments, the shape of the engagement structures 103 can be varied along the length of the housing 102 to provide varying types, frequencies, and/or strengths of tactile or audible feedback and/or to increase the degree of resistance to the operator's force required to be exerted to move the slider component.

For example, with regard to the degree of resistance provided by the engagement structures 103, in some embodiments, the engagement structures 103 can be configured to require the operator to overcome a successively higher degree of resistance as the shunt is being exposed and eventually released from the inserter. Thus, the size (e.g., height or axial length) of the engagement structures 103 can increase in a distal direction to thereby create an increasing degree of resistance against the distal advancement of the slider component.

The engagement structures 103 can define at least one notch, serration, slot, protrusion, bump, or other modified surface to provide tactile and/or audible signals or feedback to the operator. Referring to Figures 6A-6C, various features of the engagement structures 103 are shown. As shown in Figure 6A, the engagement structure 103 can include one or more notches, serrations, slots, protrusions, or bumps having an outer or cross-sectional profile 502 that can comprise deflection-facilitating surfaces and/or anti-reversing surfaces. The radius 504 and the spacing 506 of the engagement structures 103 can be altered. In particular, the radius 504 of the engagement structures 103 can be altered to provide stronger feedback or to resist motion of the slider component 106.

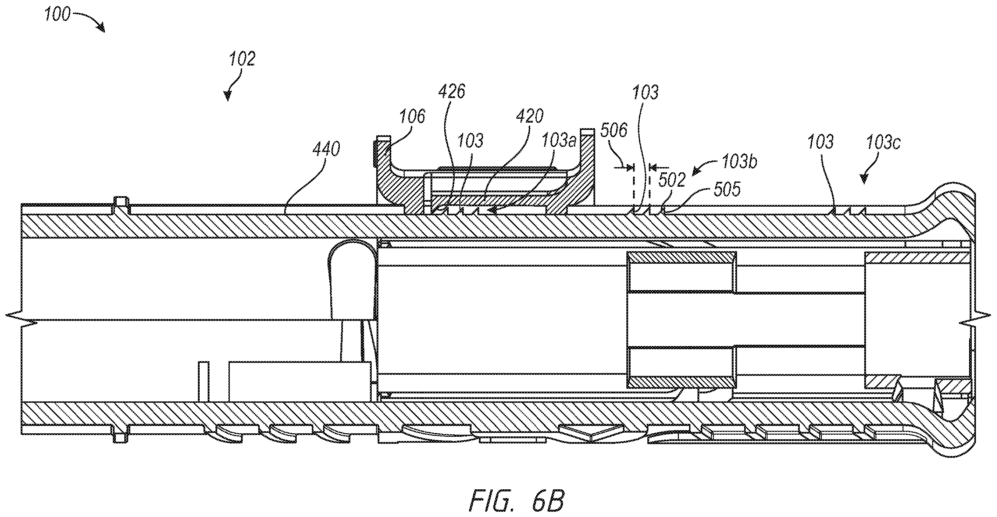

As shown in Figure 6B, the engagement structure 103 can include a perpendicular distal-facing surface 505 that provides a substantial drop from the tip or height of the engagement structure 103. In some embodiments, the surface 505 can provide an auditory function as the friction tab 420 is permitted to ride up the profile 502 on a leading or proximal side and then, the friction tab 420 springs, snaps, or clicks downwardly or radially inwardly against the housing 102 to provide an audible and/or tactile signal.

In accordance with some embodiments, the radius or angle of the profile 502 or the height of the engagement structure 103 can be modified to provide a different sound, tactile signal, or to increase sliding resistance against the slider component 106 as the slider component 106 is across the engagement structure 103.

Optionally, when the engagement structures 103 are grouped together, the spacing between the engagement structures 103 can be altered to change the frequency of occurrence of the audible signals from the auditory mechanism of the friction tab 420.

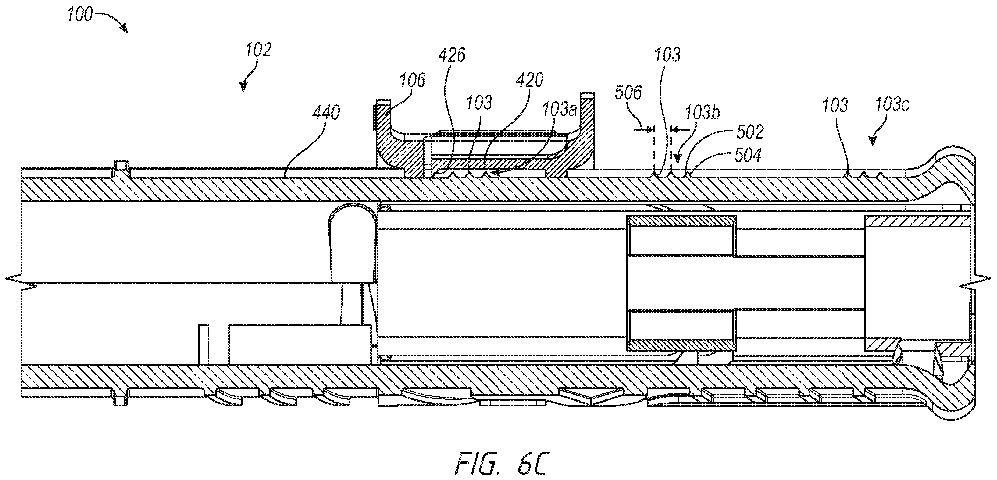

As shown in Figure 6C, the engagement structures 103 can comprise a cross-sectional profile 502 having tapered peaks. The tapered peaks may provide a different audible and/or tactile feedback compared to the features of the bumps or serrated structures shown in Figures 6A and 6B. Similar to the structures illustrated in Figures 6A and 6B, the height and the spacing of the tapered peaks can be altered to provide a desired audible or tactile signal.

In some embodiments, different engagement structures 103 can utilize different features to provide different signals to an operator. In some embodiments, a single engagement structure 103 can utilize a combination of the features described in Figures 6A-6C.

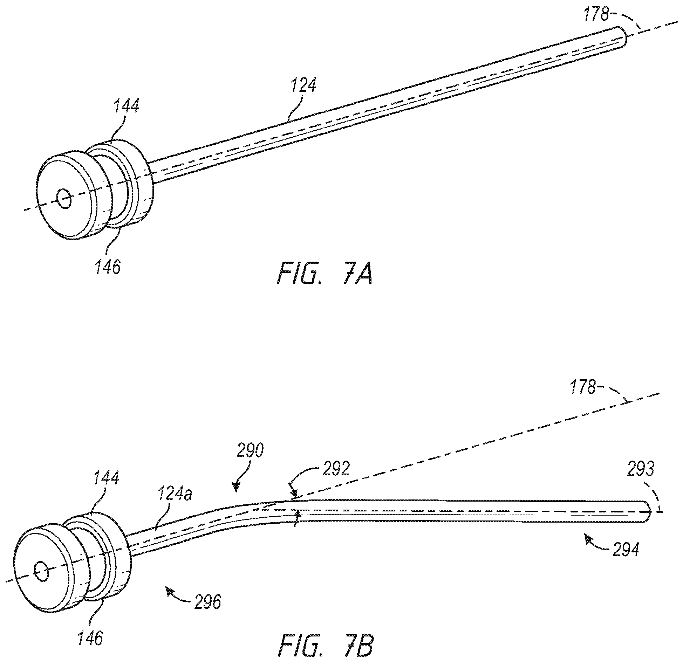

As illustrated, Figure 7A is a perspective view of a sleeve mount 144 coupled to a straight sleeve component 124, as also shown and discussed above in the embodiment of Figure 2. However, the sleeve component can also be configured to comprise a bend, as illustrated in Figure 7B. Figure 7B illustrates a sleeve component 124a that has a slight curve or bend 290. The bend 290 can be adjacent to the sleeve mount 144 and provide an angular deviation 292 of an axis 293 of the sleeve component 124a from the longitudinal axis 178 within a range of between about 3 degrees to about 30 degrees, between about 4 degrees to about 15 degrees, between about 5 degrees to about 13 degrees, or of about 8 degrees relative to the longitudinal axis of the inserter 100.

The bend in the sleeve component 124a can improve the accessibility to areas of the eye, such as when the inserter approaches the eye from a position in which the inserter is positioned above the cheekbone.

Additionally, as illustrated, an insertion or distal end portion 294 of the sleeve component 124a can be substantially straight while a deployment or proximal end portion 296 of the sleeve component 124a can comprise a curve or bend. Further, in some embodiments, the distal end portion 294 and the proximal end portion 296 can both comprise a bend or be straight with a bend section disposed therebetween. The proximal end portion 296 can be about a quarter to about a half of the overall length of the sleeve component 124a. In some embodiments, the length of the proximal end portion 296 can be about one third of the length of the sleeve component 124a. Accordingly, in some embodiments, the distal end portion 294 can be about a half to about three quarters of the length of the sleeve component 124a, and in some embodiments, about two thirds of the length of the sleeve component 124a. Advantageously then, the distal end portion 294 of the sleeve component 124a can be of a sufficient length such that the entirety of the sleeve component 124a that enters the eye is substantially straight.

While the sleeve component 124a can comprise a rigid structure that can withstand typical bending stresses in performing embodiments of the procedures disclosed herein, the needle component 120 can be made from a flexible shaft that can deflect during proximal withdrawal of the needle component 120 into the sleeve component 124a.

Thus, a proximal portion of the needle component 120 that extends along the bend 290 of the sleeve component 124a can be proximally withdrawn into the sleeve component 124a proximal or adjacent to the sleeve mount 144. After such motion, although the proximal portion of the needle component 120 was bended, that same portion of the needle component 120 can flex and straighten out as the needle component 120 is pulled proximally into a straight portion of the needle component 120 or other components within the inserter. Additionally, portions of the needle component 120 that reside in the distal end portion of the sleeve component 124a (and are therefore in a straight configuration) can be flexed or deflected into a curved or bended configuration when the needle component 120 is proximally retracted through the bend 290 of the sleeve component 124a.

Accordingly, the use of an arcuate or bent sleeve component 124a in combination with a flexible or conforming needle component 120 can allow some embodiments of the inserter to provide improved accessibility to areas of the eye.

Some embodiments can implement aspects of the sleeve structures and methods of use disclosed in applicant's U.S. Patent Application Publ. No. 2012/0123434, the entirety of which is incorporated herein by reference.

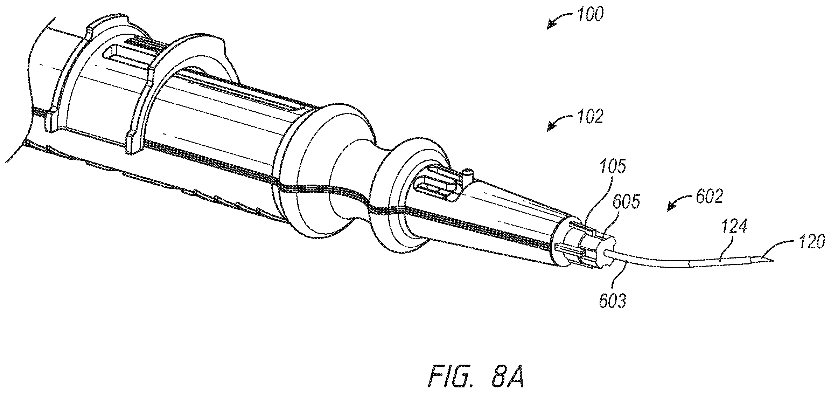

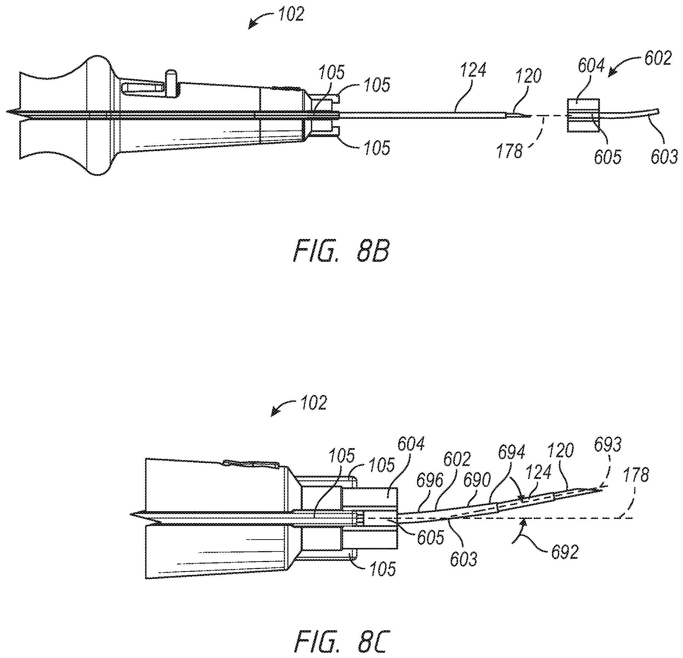

Referring to Figure 8A-10A, in some embodiments, it may be desirable that the shaft or needle component 120 comprise a bend for some of the reasons discussed herein. In some embodiments, the bend can be between about 1 degree and about 20 degrees, about 2 degrees and about 18 degrees, about 3 degrees and about 16 degrees, about 4 degrees and about 14 degrees, about 3 degrees and about 16 degrees, about 5 degrees and about 12 degrees, about 6 degrees and about 10 degrees, or about 1 degrees, about 2 degrees, about 3 degrees, about 4 degrees, about 5 degrees, about 6 degrees, about 7 degrees, about 8 degrees, about 9 degrees, about 10 degrees, about 11 degrees, about 12 degrees, about 13 degrees, about 14 degrees, about 15 degrees, about 16 degrees, about 17 degrees, about 18 degrees, about 19 degrees, or about 20 degrees.

Optionally, in some embodiments, the needle component 120 can be held in a bended configuration. In accordance with some embodiments, the sleeve component 124 can be straight and/or selectively angled or bent with the use of a removable or retrofittable end component, deflector component, or alignment guide 602. In some embodiments, an inserter 100 can be delivered with an alignment guide 602 coupled to the inserter 100 or disposed over the sleeve component 124.

Referring to Figure 8A, the alignment guide 602 can comprise a hollow guide shaft 603 that is coupled to an attachment portion 604. The attachment portion 604 can be keyed or indexed in order to rotationally orient the alignment guide 602 relative to the housing 102 of the inserter 100. For example, the attachment portion 604 can serve to couple the alignment guide 602 to the housing 102 in a desired angular or rotational orientation in order to set a bend direction and/or of the needle relative to the longitudinal axis of the housing 102 of the inserter 100.

In some embodiments, the hollow guide shaft 603 can be disposed over portions of the sleeve component 124 and the needle component 120. The guide shaft 603 can have an angle similar to or determine the angle of the angled sleeve component described herein. For example, the alignment guide 602 can bend the sleeve component 124 and the needle component 120 at a bend 690 and provide an angular deviation 692 of an axis 693 of the guide shaft 603 from a longitudinal axis 178 of the inserter 100 within a range of between about 0 degrees and about 30 degrees, between about 0 degrees and about 20 degrees, between about 0 degrees and about 15 degrees, or at about 8 degrees relative to the longitudinal axis of the inserter.

Thus, in some embodiments, an operator can modify a needle of an inserter by applying the alignment guide to the inserter, thereby bending the needle to a desired angular orientation. The alignment guide can be provided as part of a set of alignment guides that have different angular orientations. The alignment guide can be retrofittable to any existing inserter. Further, the alignment guide can be configured to mate with distal end portion of inserter in order to securely engage the alignment guide rotationally and longitudinally relative to the inserter.

For example, in some embodiments, the operator can rotate the needle until the bevel begins to push the conjunctiva away from the sclera, as discussed and shown in U.S. Patent No. 9,585,790, which is referred to herein. This procedure, which can be referred to as "tenting" the conjunctiva, can create a small space or gap between the conjunctiva and the sclera adjacent to the bevel of the needle. Once a space has been created by tenting the conjunctive, a shunt can be advanced into the space from the needle. As a result, the shunt can be substantially easier to push into the space because the conjunctiva has been pushed away and is not immediately obstructing the advancement of the shunt into the subconjunctival space.

Additionally, in some embodiments, an insertion or distal end portion 694 of the guide shaft 603 can be substantially straight while a deployment or proximal end portion 696 of the guide shaft 603 can comprise a curve or bend. Further, in some embodiments, the distal end portion 694 and the proximal end portion 696 can both comprise a bend or be straight with a bend section disposed therebetween. The proximal end portion 696 can be about one quarter to about one half of the overall length of the guide shaft 603. In some embodiments, the length of the proximal end portion 696 can be about one third of the length of the guide shaft 603. Accordingly, in some embodiments, the distal end portion 694 can be about one half to about three quarters of the length of the guide shaft 603, and in some embodiments, about two thirds of the length of the guide shaft 603.

The alignment guide 602 can allow an operator to modify the angle of the sleeve component 124 and the needle component 120 prior to a procedure (e.g., by permitting the operator to select from a variety of different alignment guides having different angular orientations and configurations of relative lengths of the proximal and distal end portions) without having to replace the needle component 120 of the inserter 100. Further, the guide shaft 603 can provide enhanced stiffness to the sleeve component 124 and the needle component 120. In some embodiments, the alignment guide 602 can facilitate the use of thinner gauge needles for the needle component 120, including, but not limited to needles of 28 Gauge or thinner in size. Thus, implementations of the present disclosure can advantageously allow very small, delicate needles to be used in the delivery of an intraocular shunt while ensuring that the needle exhibits sufficient strength and stiffness during the delivery process.

The sleeve component 124 and the needle component 120 can be flexible or elastic to allow deflection when the alignment guide 602 is installed. The alignment guide 602 can be removed to allow the sleeve component 124 and the underlying needle component 120 to move to a default straight configuration. For example, the alignment guide 602 can be configured to elastically deform the sleeve component 124. Thus, upon removal of the alignment guide 602, the sleeve component 124 and the needle component 120 will return to a straight configuration. Further, the alignment guide 602 can be reinstalled on the housing 102, if needed.

As shown in Figures 8A-8C, in some embodiments, proper rotational alignment of the alignment guide 602 can be facilitated by the attachment portion 604, which can be keyed or indexed, that orients the alignment guide 602 relative to the housing 102. The index grooves 605 of the attachment portion 604 can align with the index protrusions 105 of the housing 102. In some embodiments, the index grooves 605 can be keyed to the index protrusions 105 to allow the alignment guide 602 to attach to the housing 102 in a desired orientation. Thus, the alignment guide 602 and the inserter 100 can be configured to have one or more preset relative orientations. The index grooves 605 can be in the shape of longitudinally extending indentations or slots formed in the attachment portion 604.

Further, the index grooves 605 can be spaced apart from each other (e.g., circumferentially) at equal circumferential distances, and the index protrusions 105 can be spaced apart from each other (e.g., circumferentially) at equal circumferential spacings, so that the alignment guide 602 can be rotated to one or more preset rotational orientations. However, the circumferential distances between the index grooves 605 and/or the index protrusions 105 can vary. In the embodiment illustrated in Figures 8A-8C, there are four preset rotational orientations. In some embodiments, the alignment guide 602 can comprise a single index groove 605 that can be mated with a single index protrusion 105 of the housing 102 so that the alignment guide 602 has a single rotational orientation relative to the inserter 100.

The attachment portion 604 can have a number of index grooves 605 that is the same in number as the index protrusions 105. However, in some embodiments, the alignment guide 602 can comprise more index grooves 605 than there are index protrusions 105. For example, although there can be four index protrusions 105 and four index grooves 605, there can be four index protrusions 105 and eight index grooves 605, four index protrusions 105 and twelve index grooves 605, or ratios of index protrusions 105 to index grooves 605 of 1:4, 1:5, 1:6, or more.

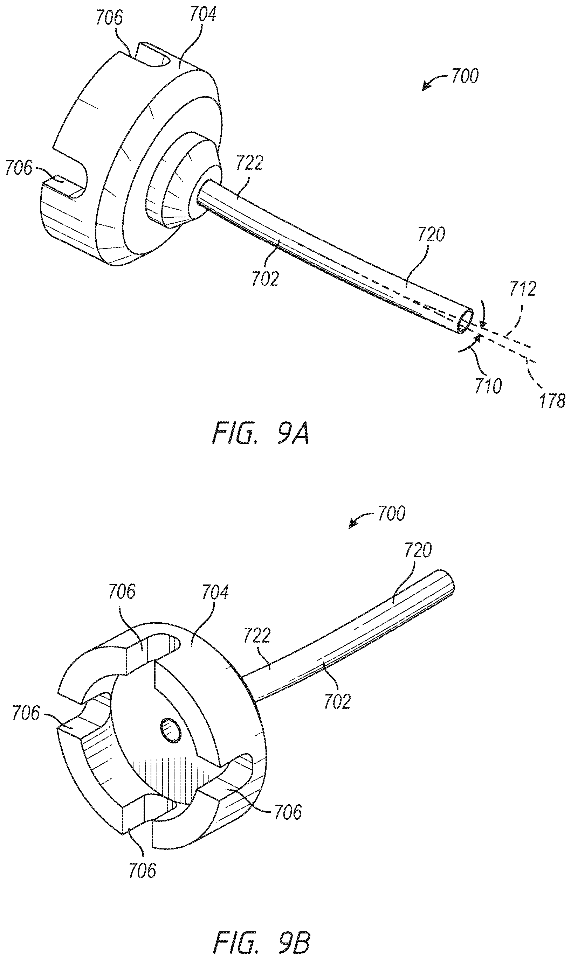

Figure 9A is a front perspective view of another retrofittable end component, deflector component, or alignment guide 700, according to some embodiments. Similar to the alignment guide 602 shown in Figures 8A-8C, the alignment guide 700 can be used to bend or maintain the sleeve component 124 in a straight and/or selectively angled or bent configuration. Certain details or usage of the alignment guide 602 can also be implemented with the alignment guide 700, as discussed herein, and will not be repeated here for brevity.

As shown in Figures 9A and 9B, The alignment guide 700 can comprise a guide shaft 702 that is coupled to an attachment portion 704. Similar to the alignment guide 602, the attachment portion 704 can comprise one or more index grooves 706 that facilitate alignment and/or coupling of the alignment guide 700 relative to the housing 102 of the inserter 100.

As with the alignment guide 602 discussed above, the inserter 100 can be delivered with the alignment guide 700 coupled to the inserter 100 or disposed over the sleeve component 124. In some embodiments, the hollow guide shaft 702 can be disposed over portions of the sleeve component 124 and the needle component 120. The guide shaft 702 can have an angle similar to or determine the angle of the angled sleeve component described herein. The alignment guide 700 can bend the sleeve component 124 and the needle component 120 and provide an angular deviation 710 of an axis 712 of the guide shaft 702 from a longitudinal axis 178 of the inserter 100 within a range of between about 0 degrees and about 30 degrees, between about 0 degrees and about 20 degrees, between about 0 degrees and about 15 degrees, or at about 8 degrees relative to the longitudinal axis of the inserter.

Additionally, similar to the alignment guide 602, an insertion or distal end portion 720 of the guide shaft 702 can be substantially straight while a deployment or proximal end portion 722 of the guide shaft 702 can comprise a curve or bend. Further, in some embodiments, the distal end portion 720 and the proximal end portion 722 can both comprise a bend or be straight with a bend section disposed therebetween. The proximal end portion 722 can be about one quarter to about one half of the overall length of the guide shaft 702. In some embodiments, the length of the proximal end portion 722 can be about one third of the length of the guide shaft 702. Accordingly, in some embodiments, the distal end portion 720 can be about one half to about three quarters of the length of the guide shaft 702, and in some embodiments, about two thirds of the length of the guide shaft 702.

Similar to the alignment guide 602, the alignment guide 700 can allow an operator to modify the angle of the sleeve component 124 and the needle component 120 prior to a procedure (e.g., by permitting the operator to select from a variety of different alignment guides having different angular orientations and configurations of relative lengths of the proximal and distal end portions) without having to replace the needle component 120 of the inserter 100. Further, the guide shaft 702 can provide enhanced stiffness to the sleeve component 124 and the needle component 120. In some embodiments, the alignment guide 700 can facilitate the use of thinner gauge needles for the needle component 120, including, but not limited to needles of 28 Gauge or thinner in size. Thus, implementations of the present disclosure can advantageously allow very small, delicate needles to be used in the delivery of an intraocular shunt while ensuring that the needle exhibits sufficient strength and stiffness during the delivery process.

As also similarly noted above, the sleeve component 124 and the needle component 120 can be flexible or elastic to allow deflection when the alignment guide 700 is installed. The alignment guide 700 can be removed to allow the sleeve component 124 and the underlying needle component 120 to move to a default straight configuration. For example, the alignment guide 700 can be configured to elastically deform the sleeve component 124. Thus, upon removal of the alignment guide 700, the sleeve component 124 and the needle component 120 will return to a straight configuration. Further, the alignment guide 700 can be reinstalled on the housing 102, if needed.

As noted similarly above with respect to Figures 8A-8C, the alignment guide 700 of Figures 9A and 9B can be properly rotationally aligned relative to the inserter 100 by the attachment portion 704, which can be keyed or indexed, that orients the alignment guide 700 relative to the housing 102. The index grooves 706 of the attachment portion 704 can align with the index protrusions 105 of the housing 102. In some embodiments, the index grooves 706 can be keyed to the index protrusions 105 to allow the alignment guide 700 to attach to the housing 102 in a desired orientation. Thus, the alignment guide 700 and the inserter 100 can be configured to have one or more preset relative orientations. The index grooves 706 can be in the shape of longitudinally extending indentations or slots formed in the attachment portion 704.

Further, the index grooves 706 can be spaced apart from each other (e.g., circumferentially) at equal circumferential distances, and the index protrusions 105 can be spaced apart from each other (e.g., circumferentially) at equal circumferential spacings, so that the alignment guide 700 can be rotated to one or more preset rotational orientations. However, the circumferential distances between the index grooves 706 and/or the index protrusions 105 can vary. In the embodiment illustrated in Figures 9A and 9B, there are four preset rotational orientations. In some embodiments, the alignment guide 700 can comprise a single index groove 706 that can be mated with a single index protrusion 105 of the housing 102 so that the alignment guide 700 has a single rotational orientation relative to the inserter 100.

The attachment portion 704 can have a number of index grooves 706 that is the same in number as the index protrusions 105. However, in some embodiments, the alignment guide 700 can comprise more index grooves 706 than there are index protrusions 105. For example, although there can be four index protrusions 105 and four index grooves 706, there can be four index protrusions 105 and eight index grooves 706, four index protrusions 105 and twelve index grooves 706, or ratios of index protrusions 105 to index grooves 706 of 1:4, 1:5, 1:6, or more.

In accordance with some embodiments, the attachment portion 704 of the alignment guide 700 can comprise one or more retention or engagement features that enable the alignment guide to snap onto or otherwise engage with corresponding engagement features of the distal end portion of the inserter 100. Such features can also be used in conjunction with the attachment portion 604 of the alignment guide 602.

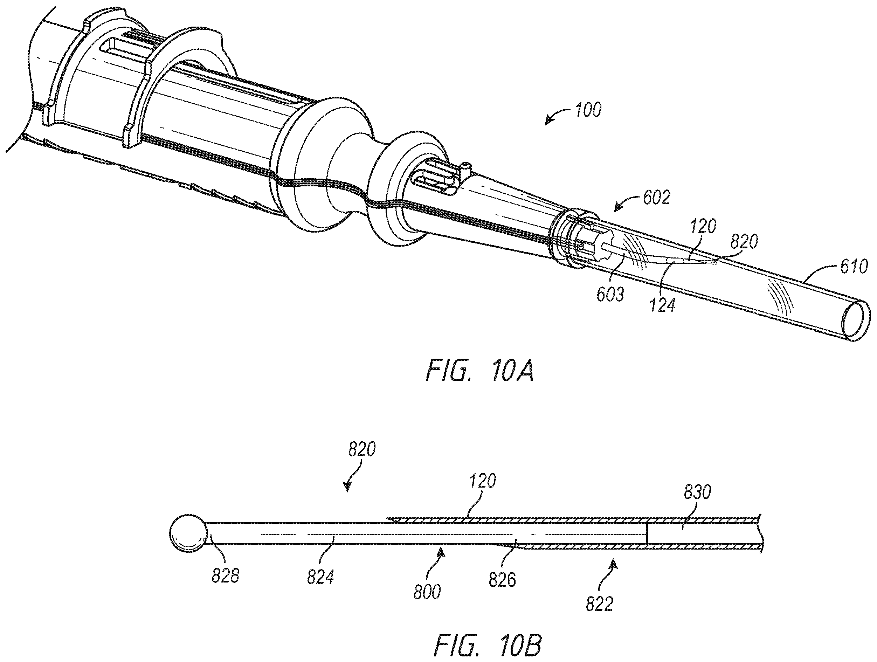

In accordance with some embodiments, various components can be used to protect the needle component of the inserter. These components can be used individually or in combination with each other to reposition and/or protect the needle component, such as the bevel of the needle component, from being damaged during transport or shipping of the inserter or the needle assembly. Such components that can be used for this purpose include the alignment guide 602 or 700, a protective cap, and a bevel protection device. These components and examples of their combined uses are discussed below with regard to Figures 10A and 10B.

As shown in Figures 10A and 10B, in some embodiments, a bevel protection device 820 can be inserted into the needle component 120 in order to protect a bevel area or bevel 800 of the needle component 120. As illustrated in Figure 10A, in some embodiments, the alignment guide 602 (which can also be the alignment guide 700) can be coupled to the inserter 100 and used to angle the sleeve component 124 and/or needle component 120 to protect the sleeve component 124 and/or needle component 120 by keeping the sleeve component 124 angled towards the protective cap 610 while the bevel protection device 820 is inserted into the needle component 120. Thus, as illustrated, the bevel protection device 820 can extend distally from the needle component 120 and contact the inner sidewall of the protective cap 610. Therefore, with the alignment guide 602 bending the needle component 120 in a direction away from the central axis of the protective cap 610 (or toward a sidewall of the protective cap 610), the bevel protection device 820 can be configured to contact the sidewall of the protective cap 610, thus spacing the bevel 800 of the needle component 120 away from and avoiding contact with the sidewall of the protective cap 610.

Additionally, the protective cap 610 configured to engage with a portion of the housing 102 in order to secure the protective cap 610 onto a distal portion of the housing 102 in order to cover and protect the sleeve component 124 and the needle component 120.

As noted above, in accordance with some embodiments, the bevel protection device 820 can also be used to reduce or prevent inadvertent contact of the bevel 800 of the needle component with other structures, such as the protective cap 610, during transport and shipping of the inserter or the needle assembly. When used in combination with the alignment guide 602 or 700, the alignment guide 602 or 700 can cause a desired contact between the bevel protection device 820 and the protective cap 610 to position the needle component 120 in a protected position. However, in some embodiments, the bevel protection device 820 can be used with by itself or with either or both of the protective cap 610 or the alignment guide 602 or 700.

The inserter 100 can be used in combination with a bevel protection device that engages with a needle component 120 of the inserter 100 in order to prevent accidental damage to the bevel 800 of the needle component 120. In some embodiments, the bevel protection device described herein can be used with the angled sleeve component 124 and/or the alignment guide 602 or 700 to dispose an end of the protection device against the protective cap 610.

For example, Figure 10B illustrates a distal end portion of a needle component 120 of an inserter. The bevel protection device 820 can engage with a distal end portion 822 of the needle component 120. The bevel protection device 820 can comprise an elongate body 824 that comprises a first portion 826 and a second portion 828. The first portion 826 can taper from a larger diameter cross-section to a smaller diameter cross-section. The smaller diameter cross-section can be less than an inner diameter of the distal end portion 822 of the needle component 120. Thus, the first portion 826 can be inserted into a lumen 830 of the needle component 120.

The elongate body 824 can be configured such that the tapering of the first portion 826 provides the elongate body 824 with a variable diameter cross-section. The diameter can taper gradually or in steps.

As shown in the embodiment illustrated in Figure 10B, the cross-sectional profile or diameter of the elongate body 824 adjacent to the second portion 828 can be greater than the cross-sectional profile or diameter of the elongate body 824 near the first portion 826. For example, from the first portion 826 toward the second portion 828, the cross-sectional diameter of the elongate body 824 can increase from a diameter that is less than an inner diameter of the lumen 830 of the needle component 120 to a diameter that is greater than the inner diameter of the lumen 830. Thus, the elongate body 824 can be inserted into the lumen 830 of the needle component 120 and advanced to a position at which the cross-section of the elongate body is about equal to the inner diameter of the lumen 830, thus restricting further advancement of the bevel protection device 820 into the lumen 830.

In some embodiments, the elongate body 824 can frictionally engage with the distal end portion 822 of the needle component 120. For example, the retention device 820 can be force fit into the needle component 120 to create a frictional engagement between the outer surface of the elongate body 824 and an inner surface of the lumen 830. This frictional engagement can be overcome by exerting a withdrawal force on the second portion 828 of the retention device 820, thereby pulling the bevel protection device 820 out of the lumen 830.

Although the bevel protection device 820 is illustrated as having a circular or diametrical cross section, other cross sections can also be used, such as triangular, square, rectangular, polygonal, star-shaped, or other similar profiles. Further, the bevel protection device 820 can be made of steel. In accordance with some embodiments, the bevel protection device 820 may only contact the inside of the needle bevel 800, and therefore advantageously does not affect the needle sharpness, which is driven by the needle outside edges.

The bevel protection device 820 can therefore ensure that the edges of the bevel 800 of the needle to not come into contact with other surfaces to prevent damage during shipment or initial handling of the inserter or needle assembly. When the operator is prepared to use in inserter, the bevel protection device 820 can be withdrawn from the needle component 120 and the procedure can be carried out.

Further, in some embodiments, the inserter 100 can comprise tactile or audible feedback mechanisms that do not require or generate consistent or persistent frictional engagement against the housing 102. Thus, features of the inserter discussed herein can be incorporated into some embodiments while excluding other features discussed herein.

Although the detailed description contains many specifics, these should not be construed as limiting the scope of the subject technology but merely as illustrating different examples and aspects of the subject technology. It should be appreciated that the scope of the subject technology includes other embodiments not discussed in detail above. Various other modifications, changes and variations may be made in the arrangement, operation and details of the method and apparatus of the subject technology disclosed herein without departing from the scope of the present disclosure. Unless otherwise expressed, reference to an element in the singular is not intended to mean "one and only one" unless explicitly stated, but rather is meant to mean "one or more." In addition, it is not necessary for a device or method to address every problem that is solvable by different embodiments of the disclosure in order to be encompassed within the scope of the disclosure.

A reference to an element in the singular is not intended to mean one and only one unless specifically so stated, but rather one or more. For example, "a" module may refer to one or more modules. An element proceeded by "a," "an," "the," or "said" does not, without further constraints, preclude the existence of additional same elements.

Headings and subheadings, if any, are used for convenience only and do not limit the invention. The word exemplary is used to mean serving as an example or illustration. To the extent that the term include, have, or the like is used, such term is intended to be inclusive in a manner similar to the term comprise as comprise is interpreted when employed as a transitional word in a claim. Relational terms such as first and second and the like may be used to distinguish one entity or action from another without necessarily requiring or implying any actual such relationship or order between such entities or actions.

Phrases such as an aspect, the aspect, another aspect, some aspects, one or more aspects, an implementation, the implementation, another implementation, some implementations, one or more implementations, an embodiment, the embodiment, another embodiment, some embodiments, one or more embodiments, a configuration, the configuration, another configuration, some configurations, one or more configurations, the subject technology, the disclosure, the present disclosure, other variations thereof and alike are for convenience and do not imply that a disclosure relating to such phrase(s) is essential to the subject technology or that such disclosure applies to all configurations of the subject technology. A disclosure relating to such phrase(s) may apply to all configurations, or one or more configurations. A disclosure relating to such phrase(s) may provide one or more examples. A phrase such as an aspect or some aspects may refer to one or more aspects and vice versa, and this applies similarly to other foregoing phrases.

A phrase "at least one of" preceding a series of items, with the terms "and" or "or" to separate any of the items, modifies the list as a whole, rather than each member of the list. The phrase "at least one of" does not require selection of at least one item; rather, the phrase allows a meaning that includes at least one of any one of the items, and/or at least one of any combination of the items, and/or at least one of each of the items. By way of example, each of the phrases "at least one of A, B, and C" or "at least one of A, B, or C" refers to only A, only B, or only C; any combination of A, B, and C; and/or at least one of each of A, B, and C.

It is understood that the specific order or hierarchy of steps, operations, or processes disclosed is an illustration of exemplary approaches. Unless explicitly stated otherwise, it is understood that the specific order or hierarchy of steps, operations, or processes may be performed in different order. Some of the steps, operations, or processes may be performed simultaneously. The accompanying method claims, if any, present elements of the various steps, operations or processes in a sample order, and are not meant to be limited to the specific order or hierarchy presented. These may be performed in serial, linearly, in parallel or in different order. It should be understood that the described instructions, operations, and systems can generally be integrated together in a single software/hardware product or packaged into multiple software/hardware products.

In one aspect, a term coupled or the like may refer to being directly coupled. In another aspect, a term coupled or the like may refer to being indirectly coupled.

Terms such as top, bottom, front, rear, side, horizontal, vertical, and the like refer to an arbitrary frame of reference, rather than to the ordinary gravitational frame of reference. Thus, such a term may extend upwardly, downwardly, diagonally, or horizontally in a gravitational frame of reference.

The disclosure is provided to enable any person skilled in the art to practice the various aspects described herein. In some instances, well-known structures and components are shown in block diagram form in order to avoid obscuring the concepts of the subject technology. The disclosure provides various examples of the subject technology, and the subject technology is not limited to these examples. Various modifications to these aspects will be readily apparent to those skilled in the art, and the principles described herein may be applied to other aspects.

The present invention is defined by the appended claims.

## Claims

1. A system for deploying an intraocular shunt, the system comprising:
an intraocular shunt inserter (100) comprising a housing (102) having a distal end portion (294) and a needle (120) extending from the distal end portion (294); and
a deflector component (602) releasably attachable to the distal end portion (294) of the intraocular shunt inserter (100), the deflector component (602) having a needle guide (603) configured to receive the needle (120) of the intraocular shunt inserter (100) therein, wherein the needle guide (603) maintains the needle (120) in a bent configuration,
**characterized in that**
the deflector component (602) comprises an alignment guide (602), the alignment guide comprising index grooves aligned with corresponding index protrusions on the housing (102) to allow the alignment guide (602) to attach to the housing (102) in a desired orientation and define a rotational orientation of the needle guide (603) relative to the intraocular shunt inserter (100).

2. The system of Claim 1, wherein the needle guide (603) comprises a hollow shaft.

3. The system of Claim 1, wherein, in the bent configuration, the needle (120) is bent at an angle of between 6 degrees to 10 degrees.

4. The system of claim 1, wherein the distal end portion (294) of the intraocular shunt inserter (100) comprises an indexing structure forming the index protrusions and the deflector component (602) comprises an alignment index forming the index grooves, wherein the alignment index of the deflector component (602) can be releasably engaged with the indexing structure to define a rotational orientation of the deflector component (602) relative to the intraocular shunt inserter (100).

5. The system of claim 4, wherein the deflector component (602) comprises a coupler (604), the needle guide (603) being attached to the coupler (604), wherein the alignment index is formed along the coupler (604).

6. The system of Claim 5, wherein the alignment index comprises at least one index groove extending along a perimeter of the coupler (604).

7. The system of Claim 4, wherein the indexing structure comprises at least one index protrusion configured to slide into a corresponding groove.

8. The system of Claim 1, wherein the distal end portion (294) of the housing (102) has at least one of the index protrusions.

9. The system of claim 8, wherein the deflector component (602) has an attachment portion (604) releasably attachable to the distal end portion (294) of the intraocular shunt inserter (100), the attachment portion (604) having a longitudinally extending groove configured to receive the index protrusion to engage the deflector component (602) with the intraocular shunt inserter (100) at a predetermined angle around a longitudinal axis (178).

10. The system of claim 9, wherein the attachment portion (604) comprises a cavity wall defining a cavity configured to receive the distal end portion (294) of the intraocular shunt inserter (100), and the cavity wall defines a longitudinally extending slot.

11. The system of Claim 8, wherein the needle guide (603) comprises a hollow shaft.

12. The system of Claim 8, wherein, in the bent configuration, the needle (120) is bent at an angle of between 6 degrees to 10 degrees.

13. The system of claim 8, wherein the distal end portion (294) of the intraocular shunt inserter (100) comprises an indexing structure forming the index protrusions and the deflector component (602) comprises an alignment index forming the index grooves, wherein the alignment index of the deflector component (602) can be releasably engaged with the indexing structure to define a rotational orientation of the deflector component (602) relative to the intraocular shunt inserter (100).

14. The system of claim 13, wherein the distal end portion (294) comprises a rib extending radially from the distal end portion (294), and the longitudinally extending slot is configured to receive the rib of the distal end portion (294).

15. The system of claim 8, wherein the deflector component (602) can be releasably engaged with the index protrusion to define a second rotational orientation of the deflector component (602) relative to the intraocular shunt inserter (100).

## Patentansprüche

1. Ein System zum Einsetzen eines intraokularen Shunts, wobei das System umfasst:
ein Intraokularshunt-Einführer (100) mit einem Gehäuse (102), das einen distalen Endabschnitt (294) und eine Nadel (120) aufweist, die sich vom distalen Endabschnitt (294) erstreckt; und
eine Ablenkungskomponente (602), die lösbar an dem distalen Endabschnitt (294) des Intraokularshunt-Einführers (100) befestigbar ist, wobei die Ablenkungskomponente (602) eine Nadelführung (603) aufweist, die dazu konfiguriert ist, die Nadel (120) des intraokularen Shunt-Einführers (100) aufzunehmen, wobei die Nadelführung (603) die Nadel (120) in einer gebogenen Konfiguration hält,
**dadurch gekennzeichnet, dass**
die Ablenkungskomponente (602) eine Ausrichtungsführung (602) umfasst, wobei die Ausrichtungsführung Indexnuten umfasst, die mit entsprechenden Indexvorsprüngen am Gehäuse (102) ausgerichtet sind, damit die Ausrichtungsführung (602) in einer gewünschten Ausrichtung am Gehäuse (102) befestigt werden kann und eine Drehausrichtung der Nadelführung (603) relativ zum Intraokularshunt-Einführer (100) definiert.

2. Das System nach Anspruch 1, wobei die Nadelführung (603) einen Hohlschaft umfasst.

3. Das System nach Anspruch 1, wobei die Nadel (120) in der gebogenen Konfiguration in einem Winkel zwischen 6 Grad und 10 Grad gebogen ist.

4. Das System nach Anspruch 1, wobei der distale Endabschnitt (294) des IntraokularShunt-Einführers (100) eine Indexierungsstruktur umfasst, die die Indexvorsprünge bildet, und die Ablenkungskomponente (602) einen Ausrichtungsindex umfasst, der die Indexnuten bildet, wobei der Ausrichtungsindex der Ablenkungskomponente (602) lösbar mit der Indexierungsstruktur in Eingriff gebracht werden kann, um eine Drehausrichtung der Ablenkungskomponente (602) relativ zum Intraokularshunt-Einführer (100) zu definieren.

5. Das System nach Anspruch 4, wobei die Ablenkungskomponente (602) einen Koppler (604) umfasst, wobei die Nadelführung (603) an dem Koppler (604) befestigt ist, wobei der Ausrichtungsindex entlang des Kopplers (604) ausgebildet ist.

6. System nach Anspruch 5, wobei der Ausrichtungsindex mindestens eine Indexnut umfasst, die sich entlang eines Umfangs des Kopplers (604) erstreckt.

7. Das System nach Anspruch 4, wobei die Indexierungsstruktur mindestens einen Indexvorsprung umfasst, der so konfiguriert ist, dass er in eine entsprechende Nut gleitet.

8. System nach Anspruch 1, wobei der distale Endabschnitt (294) des Gehäuses (102) mindestens einen der Indexvorsprünge aufweist.

9. Das System nach Anspruch 8, wobei die Ablenkungskomponente (602) einen Befestigungsabschnitt (604) aufweist, der lösbar an dem distalen Endabschnitt (294) des Intraokularshunt-Einführers (100) befestigt werden kann, wobei der Befestigungsabschnitt (604) eine sich in Längsrichtung erstreckende Nut aufweist, die dazu konfiguriert ist, den Indexvorsprung aufzunehmen, um die Ablenkungskomponente (602) mit dem Intraokularshunt-Einführer (100) in einem vorbestimmten Winkel um eine Längsachse (178) in Eingriff zu bringen.

10. Das System nach Anspruch 9, wobei der Befestigungsabschnitt (604) eine Hohlraumwand umfasst, die einen Hohlraum definiert, der dazu konfiguriert ist, den distalen Endabschnitt (294) des Intraokularshunt-Einführers (100) aufzunehmen, und wobei die Hohlraumwand einen sich in Längsrichtung erstreckenden Schlitz definiert.

11. Das System nach Anspruch 8, wobei die Nadelführung (603) einen hohlen Schaft umfasst.

12. Das System nach Anspruch 8, wobei die Nadel (120) in der gebogenen Konfiguration in einem Winkel zwischen 6 Grad und 10 Grad gebogen ist.

13. Das System nach Anspruch 8, wobei der distale Endabschnitt (294) des Intraokularshunt-Einführers (100) eine Indexierungsstruktur umfasst, die die Indexvorsprünge bildet, und die Ablenkungskomponente (602) einen Ausrichtungsindex umfasst, der die Indexnuten bildet, wobei der Ausrichtungsindex der Ablenkungskomponente (602) lösbar mit der Indexierungsstruktur in Eingriff gebracht werden kann, um eine Drehausrichtung der Ablenkungskomponente (602) relativ zum intraokularen Shunt-Einführinstrument (100) zu definieren.

14. Das System nach Anspruch 13, wobei der distale Endabschnitt (294) eine sich radial vom distalen Endabschnitt (294) erstreckende Rippe umfasst und der sich in Längsrichtung erstreckende Schlitz dazu konfiguriert ist, die Rippe des distalen Endabschnitts (294) aufzunehmen.

15. Das System nach Anspruch 8, wobei die Ablenkungskomponente (602) lösbar mit dem Indexvorsprung in Eingriff gebracht werden kann, um eine zweite Drehausrichtung der Ablenkungskomponente (602) relativ zum Intraokularshunt-Einführer (100) zu definieren.

## Revendications

1. Système pour déployer un shunt intraoculaire, le système comprenant :
un inséreur de shunt intraoculaire (100) comprenant un boîtier (102) ayant une partie d'extrémité distale (294) et une aiguille (120) s'étendant à partir de la partie d'extrémité distale (294) ; et
un composant déflecteur (602) pouvant être fixé de manière amovible à la partie d'extrémité distale (294) de l'inséreur de shunt intraoculaire (100), le composant déflecteur (602) comportant un guide-aiguille (603) configuré pour recevoir l'aiguille (120) de l'inséreur de shunt intraoculaire (100), dans lequel le guide-aiguille (603) maintient l'aiguille (120) dans une configuration courbée,
**caractérisé en ce que**
le composant déflecteur (602) comprend un guide d'alignement (602), le guide d'alignement comprenant des rainures d'indexation alignées avec des saillies d'indexation correspondantes sur le boîtier (102) pour permettre au guide d'alignement (602) de se fixer au boîtier (102) dans une orientation souhaitée et de définir une orientation de rotation du guide d'aiguille (603) par rapport à l'inséreur de shunt intraoculaire (100).

2. Le système selon la revendication 1, dans lequel le guide-aiguille (603) comprend une tige creuse.

3. Le système selon la revendication 1, dans lequel, dans la configuration courbée, l'aiguille (120) est courbée selon un angle compris entre 6 degrés et 10 degrés.

4. Le système selon la revendication 1, dans lequel la partie d'extrémité distale (294) de l'inséreur de shunt intraoculaire (100) comprend une structure d'indexation formant les saillies d'indexation et le composant déflecteur (602) comprend un index d'alignement formant les rainures d'indexation, dans lequel l'index d'alignement du composant déflecteur (602) peut être engagé de manière amovible avec la structure d'indexation pour définir une orientation de rotation du composant déflecteur (602) par rapport à l'inséreur de shunt intraoculaire (100).

5. Le système selon la revendication 4, dans lequel le composant déflecteur (602) comprend un coupleur (604), le guide-aiguille (603) étant fixé au coupleur (604), dans lequel l'index d'alignement est formé le long du coupleur (604).

6. Le système selon la revendication 5, dans lequel l'index d'alignement comprend au moins une rainure d'index s'étendant le long d'un périmètre du coupleur (604).

7. Le système selon la revendication 4, dans lequel la structure d'indexation comprend au moins une saillie d'indexation configurée pour coulisser dans une rainure correspondante.

8. Système selon la revendication 1, dans lequel la partie d'extrémité distale (294) du boîtier (102) comporte au moins une des saillies d'indexation.

9. Le système selon la revendication 8, dans lequel le composant déflecteur (602) comporte une partie de fixation (604) pouvant être fixée de manière amovible à la partie d'extrémité distale (294) de l'inséreur de shunt intraoculaire (100), la partie de fixation (604) comportant une rainure s'étendant longitudinalement configurée pour recevoir la saillie d'indexation afin d'engager le composant déflecteur (602) avec l'inséreur de shunt intraoculaire (100) selon un angle prédéterminé autour d'un axe longitudinal (178).

10. Le système selon la revendication 9, dans lequel la partie de fixation (604) comprend une paroi de cavité définissant une cavité configurée pour recevoir la partie d'extrémité distale (294) de l'inséreur de shunt intraoculaire (100), et la paroi de cavité définit une fente s'étendant longitudinalement.

11. Le système selon la revendication 8, dans lequel le guide-aiguille (603) comprend une tige creuse.

12. Le système selon la revendication 8, dans lequel, dans la configuration courbée, l'aiguille (120) est courbée selon un angle compris entre 6 degrés et 10 degrés.

13. Le système selon la revendication 8, dans lequel la partie d'extrémité distale (294) de l'inséreur de shunt intraoculaire (100) comprend une structure d'indexation formant les saillies d'indexation et le composant déflecteur (602) comprend un index d'alignement formant les rainures d'indexation, dans lequel l'index d'alignement du composant déflecteur (602) peut être engagé de manière amovible avec la structure d'indexation pour définir une orientation de rotation du composant déflecteur (602) par rapport à l'inséreur de shunt intraoculaire (100).

14. Le système selon la revendication 13, dans lequel la partie d'extrémité distale (294) comprend une nervure s'étendant radialement à partir de la partie d'extrémité distale (294), et la fente s'étendant longitudinalement est configurée pour recevoir la nervure de la partie d'extrémité distale (294).

15. Le système selon la revendication 8, dans lequel le composant déflecteur (602) peut être engagé de manière libérable avec la saillie d'indexation pour définir une deuxième orientation de rotation du composant déflecteur (602) par rapport à l'inséreur de shunt intraoculaire (100).
